# EUROPEAN PATENT SPECIFICATION

(11) **EP 2 040 749 B1**
(45) Date of publication and mention of the grant of the patent: **23.03.2011**
(21) Application number: 07765694.0
(22) Date of filing: 28.06.2007
(51) Int. Cl.: A61K 39/39

(54) **PHARMACEUTICAL COMPOSITIONS CONTAINING MONOCLONAL ANTI IDIOTYPIC ANTI-CA-125 ANTIBODY AND ALUMINIUM**
PHARMAZEUTISCHE ZUSAMMENSETZUNGEN MIT MONOKLONALEM ANTIIDIOTYPISCHEM ANTI-CA-125-ANTIKÖRPER UND ALUMINIUM
COMPOSITIONS PHARMACEUTIQUES CONTENANT UN ANTICORPS MONOCLONAL ANTI-IDIOTYPIQUE ANTI-CA-125 ET DE L'ALUMINIUM

(30) Priority: 29.06.2006 IT FI20060163
(43) Date of publication of application: 01.04.2009
(60) Divisional application: 10164951.5
(73) Proprietor: Menarini International Operations Luxembourg S.A., 1611 Luxembourg (LU)
(72) Inventor: FLEMMING, Jens, 22143 Hamburg (DE); GRÖGER, Karsten, 12587 Berlin (DE); SCHMITZ, Reinhard, 14055 Berlin (DE); MANZINI, Stefano, I-50121 Firenze (IT)
(74) Representative: Gervasi, Gemma
(86) International application number: PCT/EP2007/056465
(87) International publication number: WO 2008/000789

(56) References cited:
- EP-B1- 0 700 305
- GOTO N ET AL: "Local tissue irritating effects and adjuvant activities of calcium phosphate and aluminium hydroxide with different physical properties" VACCINE, BUTTERWORTH SCIENTIFIC. GUILDFORD, GB, vol. 15, no. 12-13, August 1997 (1997-08), pages 1364-1371, XP004089483 ISSN: 0264-410X
- EDELMAN R: "VACCINE ADJUVANTS" REVIEWS OF INFECTIOUS DISEASES, CHICAGO, IL, US, vol. 2, no. 3, May 1980 (1980-05), pages 370-383, XP000995807
- GUPTA R K ET AL BORCHARDT R T (ED ): "ADJUVANT PROPERTIES OF ALUMINUM AND CALCIUM COMPOUNDS" VACCINE DESIGN. SUBUNIT AND ADJUVANT APPROACH, PHARMACEUTICAL BIOTECHNOLOGY, NEW YORK, PLENUM PRESS, US, vol. VOL. 6, 1995, pages 229-248, XP002925125 ISBN: 0-306-44867-X
- BAYLOR N W ET AL: "Aluminum salts in vaccines-US perspective" VACCINE, BUTTERWORTH SCIENTIFIC. GUILDFORD, GB, vol. 20, 31 May 2002 (2002-05-31), pages S18-S23, XP004361369 ISSN: 0264-410X & BAYLOR N W ET AL: "Corrigendum to ''Aluminum salts in vaccines-US perspective'' [Vaccine 20 (Suppl. 2) (2002) S18-S23]" VACCINE, BUTTERWORTH SCIENTIFIC. GUILDFORD, GB, vol. 20, no. 27-28, 10 September 2002 (2002-09-10), page 3428, XP004378539 ISSN: 0264-410X
- GUPTA R K: "Aluminum compounds as vaccine adjuvants" ADVANCED DRUG DELIVERY REVIEWS, AMSTERDAM, NL, vol. 32, no. 3, 6 July 1998 (1998-07-06), pages 155-172, XP002231504 ISSN: 0169-409X
- REINARTZ ET AL.: "Vaccination of Patients with Advanced Ovarian Carcinoma with the Anti-Idiotype ACA125: Immunological Response and Survival (Phase Ib/II)" CLINICAL CANCER RESEARCH, vol. 10, 1 March 2004 (2004-03-01), page 1580-1587,

### SEQUENCE LISTING

<110> Menarini International operations Luxembourg S.A.
<120> Pharmaceutical compositions containing monoclonal anti idiotypic anti-CA-125 antibody and aluminium
<130> 7626 PTWO
<150> FI2006A000163
   <151> 2006-06-29
<160> 2
<170> PatentIn version 3.3
<210> 1
   <211> 443
   <212> PRT
   <213> Artificial sequence
<220>
   <223> Monoclonal antibody
<220>
   <221> MISC_FEATURE
   <222> (4)..(4)
   <223> X is Leu or Ile
<220>
   <221> MISC_FEATURE
   <222> (11)..(11)
   <223> X is Leu or Ile
<220>
   <221> MISC_FEATURE
   <222> (20)..(20)
   <223> X is Leu or Ile
<220>
   <221> MISC_FEATURE
   <222> (45)..(45)
   <223> X is Leu or Ile
<220>
   <221> MISC_FEATURE
   <222> (48)..(48)
   <223> X is Leu or Ile
<220>
   <221> MISC_FEATURE
   <222> (51)..(51)
   <223> X is Leu or Ile
<220>
   <221> MISC_FEATURE
   <222> (70)..(70)
   <223> X is Leu or Ile
<220>
   <221> MISC_FEATURE
   <222> (83)..(83)
   <223> X is Leu or Ile
<220>
   <221> MISC_FEATURE
   <222> (86)..(86)
   <223> X is Leu or Ile
<220>
   <221> MISC_FEATURE
   <222> (114)..(114)
   <223> X is Leu or Ile
<220>
   <221> MISC_FEATURE
   <222> (130)..(130)
   <223> X is Leu or Ile
<220>
   <221> MISC_FEATURE
   <222> (144)..(144)
   <223> X is Leu or Ile
<220>
   <221> MISC_FEATURE
   <222> (147)..(147)
   <223> X is Leu or Ile
<220>
   <221> MISC_FEATURE
   <222> (165)..(165)
   <223> X is Leu or Ile
<220>
   <221> MISC_FEATURE
   <222> (176)..(176)
   <223> X is Leu or Ile
<220>
   <221> MISC_FEATURE
   <222> (180)..(180)
   <223> X is Leu or Ile
<220>
   <221> MISC_FEATURE
   <222> (183)..(183)
   <223> X is Leu or Ile
<220>
   <221> MISC_FEATURE
   <222> (216)..(216)
   <223> X is Leu or Ile
<220>
   <221> MISC_FEATURE
   <222> (227)..(227)
   <223> X is Leu or Ile
<220>
   <221> MISC_FEATURE
   <222> (238)..(238)
   <223> X is Leu or Ile
<220>
   <221> MISC_FEATURE
   <222> (247)..(247)
   <223> X is Leu or Ile
<220>
   <221> MISC_FEATURE
   <222> (249)..(249)
   <223> X is Leu or Ile
<220>
   <221> MISC_FEATURE
   <222> (251)..(251)
   <223> X is Leu or Ile
<220>
   <221> MISC_FEATURE
   <222> (262)..(262)
   <223> X is Leu or Ile
<220>
   <221> MISC_FEATURE
   <222> (302)..(302)
   <223> X is Leu or Ile
<220>
   <221> MISC_FEATURE
   <222> (304)..(304)
   <223> X is Leu or Ile
<220>
   <221> MISC_FEATURE
   <222> (310)..(310)
   <223> X is Leu or Ile
<220>
   <221> MISC_FEATURE
   <222> (328)..(328)
   <223> X is Leu or Ile
<220>
   <221> MISC_FEATURE
   <222> (332)..(332)
   <223> X is Leu or Ile
<220>
   <221> MISC_FEATURE
   <222> (347)..(347)
   <223> X is Leu or Ile
<220>
   <221> MISC_FEATURE
   <222> (361)..(361)
   <223> X is Leu or Ile
<220>
   <221> MISC_FEATURE
   <222> (365)..(365)
   <223> X is Leu or Ile
<220>
   <221> MISC_FEATURE
   <222> (373)..(373)
   <223> X is Leu or Ile
<220>
   <221> MISC_FEATURE
   <222> (393)..(393)
   <223> X is Leu or Ile
<220>
   <221> MISC_FEATURE
   <222> (406)..(406)
   <223> X is Leu or Ile
<220>
   <221> MISC_FEATURE
   <222> (424)..(424)
   <223> X is Leu or Ile
<220>
   <221> MISC_FEATURE
   <222> (428)..(428)
   <223> X is Leu or Ile
<220>
   <221> MIS_FEATURE
   <222> (437)..(437)
   <223> X is Leu or Ile
<400> 1
   Gln Val Gln Xaa Gln Gln Ser Gly Ala Glu Xaa Ala Arg Pro Gly Ala
   1               5                   10                  15
   Ser Val Lys Xaa Ser Cys Lys Ala Ser Gly Tyr Thr Phe Thr Asn Tyr
               20                  25                  30
   Trp Met Gln Trp Val Lys Gln Arg Pro Gly Gln Gly Xaa Asp Trp Xaa
           35                  40                  45
   Gly Ala Xaa Tyr Pro Gly Asp Gly Asn Thr Arg Tyr Thr Gln Lys Phe
       50                  55                  60
   Lys Gly Lys Ala Thr Xaa Thr Ala Asp Lys Ser Ser Ser Thr Ala Tyr
   65                  70                  75                  80
   Met Gln Xaa Ser Ser Xaa Ala Ser Glu Asp Ser Ala Val Tyr Tyr Cys
                   85                  90                  95
   Ala Arg Gly Glu Gly Asn Tyr Ala Trp Phe Ala Tyr Trp Gly Gln Gly
               100                 105                 110
   Thr Xaa Val Thr Val Ser Ala Ala Lys Thr Thr Pro Pro Ser Val Tyr
           115                 120                 125
   Pro Xaa Ala Pro Gly Ser Ala Ala Gln Thr Asn Ser Met Val Thr Xaa
       130                 135                 140
   Gly Cys Xaa Val Lys Gly Tyr Phe Pro Glu Pro Val Thr Val Thr Trp
   145                 150                 155                 160
   Asn Ser Gly Ser Xaa Ser Ser Gly Val His Thr Phe Pro Ala Val Xaa
                   165                 170                 175
   Gln Ser Asp Xaa Tyr Thr Xaa Ser Ser Ser Val Thr Val Pro Ser Ser
               180                 185                 190
   Thr Trp Pro Ser Glu Thr Val Thr Cys Asn Val Ala His Pro Ala Ser
           195                 200                 205
   Ser Thr Lys Val Asp Lys Lys Xaa Val Pro Arg Asp Cys Gly Cys Lys
       210                 215                 220
   Pro Cys Xaa Cys Thr Val Pro Glu Val Ser Ser Val Phe Xaa Phe Pro
   225                 230                 235                 240
   Pro Lys Pro Lys Asp Val Xaa Thr Xaa Thr Xaa Thr Pro Lys Val Thr
   245 250 255
   Cys Val Val Val Asp Xaa Ser Lys Asp Asp Pro Glu Val Gln Phe Ser
   260 265 270
   Trp Phe Val Asp Asp Val Glu Val His Thr Ala Gln Thr Gln Pro Arg
   275 280 285
   Glu Glu Gln Phe Asn Ser Thr Phe Arg Ser Val Ser Glu Xaa Pro Xaa
   290 295 300
   Met His Gln Asp Trp Xaa Asn Gly Lys Glu Phe Lys Cys Arg Val Asn
   305 310 315 320
   Ser Ala Ala Phe Pro Ala Pro Xaa Glu Lys Thr Xaa Ser Lys Thr Lys
   325 330 335
   Gly Arg Pro Lys Ala Pro Gln Val Tyr Thr Xaa Pro Pro Pro Lys Glu
   340 345 350
   Gln Met Ala Lys Asp Lys Val Ser Xaa Thr Cys Met Xaa Thr Asp Phe
   355 360 365
   Phe Pro Glu Asp Xaa Thr Val Glu Trp Gln Trp Asn Gly Gln Pro Ala
   370 375 380
   Glu Asn Tyr Lys Asn Thr Gln Pro Xaa Met Asp Thr Asp Gly Ser Tyr
   385 390 395 400
   Phe Val Tyr Ser Lys Xaa Asn Val Gln Lys Ser Asn Trp Glu Ala Gly
   405 410 415
   Asn Thr Phe Thr Cys Ser Val Xaa His Glu Gly Xaa His Asn His His
   420 425 430
   Thr Glu Lys Ser Xaa Ser His Ser Pro Gly Lys
   435 440
<210> 2
   <211> 214
   <212> PRT
   <213> Artificial sequence
<220>
   <223> Monoclonal antibody
<220>
   <221> MISC_FEATURE
   <222> (21)..(21)
   <223> X is Leu or Ile
<220>
   <221> MISC_FEATURE
   <222> (29)..(29)
   <223> X is Leu or Ile
<220>
   <221> MISC_FEATURE
   <222> (33)..(33)
   <223> X is Leu or Ile
<220>
   <221> MISC_FEATURE
   <222> (46)..(47)
   <223> X is Leu or Ile
<220>
   <221> MISC_FEATURE
   <222> (54)..(54)
   <223> X is Leu or Ile
<220>
   <221> MISC_FEATURE
   <222> (73)..(73)
   <223> X is Leu or Ile
<220>
   <221> MISC_FEATURE
   <222> (75)..(75)
   <223> X is Leu or Ile
<220>
   <221> MISC_FEATURE
   <222> (78)..(78)
   <223> X is Leu or Ile
<220>
   <221> MISC_FEATURE
   <222> (85)..(85)
   <223> X is Leu or Ile
<220>
   <221> MISC_FEATURE
   <222> (94)..(94)
   <223> X is Leu or Ile
<220>
   <221> MISC_FEATURE
   <222> (104)..(104)
   <223> X is Leu or Ile
<220>
   <221> MISC_FEATURE
   <222> (106)..(106)
   <223> X is Leu or Ile
<220>
   <221> MISC_FEATURE
   <222> (117)..(117)
   <223> X is Leu or Ile
<220>
   <221> MISC_FEATURE
   <222> (125)..(125)
   <223> X is Leu or Ile
<220>
   <221> MISC_FEATURE
   <222> (136)..(136)
   <223> X is Leu or Ile
<220>
   <221> MISC_FEATURE
   <222> (144)..(144)
   <223> X is Leu or Ile
<220>
   <221> MISC_FEATURE
   <222> (150)..(150)
   <223> X is Leu or Ile
<220>
   <221> MISC_FEATURE
   <222> (160)..(160)
   <223> X is Leu or Ile
<220>
   <221> MISC_FEATURE
   <222> (179)..(179)
   <223> X is Leu or Ile
<220>
   <221> MISC_FEATURE
   <222> (181)..(181)
   <223> X is Leu or Ile
<220>
   <221> MISC_FEATURE
   <222> (205)..(205)
   <223> X is Leu or Ile
<400> 2
   Asp Ile Gln Met Thr Gln Ser Pro Ala Ser Leu Ser Ala Ser Val Gly
   1               5                   10                  15
   Glu Thr Val Thr Xaa Thr Cys Arg Ala Ser Glu Asn Xaa Tyr Ser Tyr
               20                  25                  30
   Xaa Ala Trp His Gln Gln Lys Gln Gly Lys Ser Pro Gln Xaa Xaa Val
           35                  40                  45
   Tyr Asn Ala Lys Thr Xaa Ala Gly Gly Val Ser Ser Arg Phe Ser Gly
       50                  55                  60
   Ser Gly Ser Gly Thr His Phe Ser Xaa Lys Xaa Lys Ser Xaa Gln Pro
   65                  70                  75                  80
   Glu Asp Phe Gly Xaa Tyr Tyr Cys Gln His His Tyr Gly Xaa Phe Pro
                   85                  90                  95
   Thr Phe Gly Gly Gly Thr Lys Xaa Glu Xaa Lys Arg Ala Asp Ala Ala
               100                 105                 110
   Pro Thr Val Ser Xaa Phe Pro Pro Ser Ser Glu Gln Xaa Thr Ser Gly
           115                 120                 125
   Gly Ala Ser Val Val Cys Phe Xaa Asn Asn Phe Tyr Pro Lys Asp Xaa
       130                 135                 140
   Asn Val Lys Trp Lys Xaa Asp Gly Ser Glu Arg Gln Asn Gly Val Xaa
   145                 150                 155                 160
   Asn Ser Trp Thr Asp Gln Asp Ser Lys Asp Ser Thr Tyr Ser Met Ser
                   165                 170                 175
   Ser Thr Xaa Thr Xaa Thr Lys Asp Glu Tyr Glu Arg His Asn Ser Tyr
               180                 185                 190
   Thr Cys Glu Ala Thr His Lys Thr Ser Thr Ser Pro Xaa Val Lys Ser
           195                 200                 205
   Phe Asn Arg Asn Glu Cys
       210

## Description

### Field of the application

The invention refers to the field of pharmaceutical compositions to be used as vaccine for parenteral application.

### State of the art

As it is known, in order to amplify the immunogenicity of prophylactic or therapeutic vaccines the use of adjuvant, such as an aluminium compound, is generally considered (Fiejka et al., Rocz Panstw Zakl Hig 1993, 73 - 80) in the preparation of pharmaceutical dosage forms as solutions or suspensions for parenteral administration. The target of these formulations should be inducing a maximal and long-lasting immunogenic effect. However a limited amount of aluminium adjuvants have been up to now used in registered and marketed vaccine and indeed a limit of 1.25 mg/dose is established in Ph.Eur.

Also FDA in the 21 CFR 610.15 states the amount of aluminium in the recommended individual dose of a biological product shall not exceed:
(1) 0.85 milligrams if determined by assay; or
(2) 1.14 milligrams if determined by calculation on the basis of the amount of aluminium compound added; or
(3) 1.25 milligrams determined by assay provided that data demonstrating that the amount of aluminium used is safe and necessary to produce the intended effect.

The immunogenicity of antibodies adsorbed onto aluminium adjuvants appears to depend on the degree of adsorption (Capelle et al., Vaccine 2005, 1686 - 1694), but it is unclear which is the best ratio to be used to achieve a maximal immunogenic response still maintaining a safety profile. For instance, the World Health Organisation (WHO) recommends an adsorption onto aluminium adjuvants at toxoid levels exceeding 80 % for tetanus and diphtheria toxoids (Gupta et al., Vaccine 1995, 1263 - 1276; WHO Technical Report Series No. 595, 1996, p. 6). In contrast, data show that an adsorption of tetanus components to aluminium hydroxide of levels exceeding 80 % did not enhance the immune response in healthy adults (Paoletti et al., Infect Immun 2001, 6696 - 6701). The most appropriate content of an aluminium compound to maximise the immunogenicity (without risk of unacceptable toxic side effect) of a monoclonal antibody-containing vaccine is still an open technical question.

Another technical issue is the stability of vaccines that varies considerably. They can be ranked by their resistance to storage at elevated temperatures, with diphtheria and tetanus toxoids and hepatitis B vaccine showing the highest thermostability, freeze-dried measles, yellow fever and BCG vaccine occupying the middle position and oral poliomyelitis vaccine being the most fragile (Galazka et al., Thermostability of vaccines, World Health Organisation, 1998). Reconstituted vaccines against measles, yellow fever and tuberculosis (BCG) are unstable vaccines; they should be used as soon as possible after the reconstitution and be kept in an ice bath during immunisation session. Although tetanus and diphtheria (e.g. adsorbed on aluminium salts) as monovalent vaccines or components of combined vaccines are stable for weeks at 35 -37° C, each exposure to ambient temperature results in some degradation of the vaccine (Galazka et al, vide supra , pag 48). Therefore also for these "stable" types of vaccines the storage at cold conditions is prescribed (Rote Liste, Edition Cantor Verlag, 2005).

An unsolved technical issue for this type of vaccine containing monoclonal antibodies, is the low stability at ambient temperatures.

Due to the nature of the complex protein active principle, these drug products have to be stored under cold conditions (2 - 8 °C) requiring a constant cooling chain. This causes higher distribution costs, jeopardise the overall safety profile and, possibly more important, limits the world-wide use of highly beneficial drugs like an anticancer vaccine. The development of vaccine formulations ensuring constant drug product quality even at ambient and higher temperatures (25°C - 37 °C) would therefore improve the overall safety and efficacy profile of these drugs. This would not only be highly beneficial for patients, but would also lead to an overall facilitated handling of these valuable drugs.

On the other hand the importance of immunotherpy in the treatment of tumours in humans and in particular the use of monoclonal anti idiotypic antibodies as therapeutic vaccines against tumours is well known.

Such vaccine should activate the immunity system in the host stimulating a humour and cell action against the tumour.

The development and definition of such a kind of formulation is particularly important for ACA125/MEN2234 (described in EP700305), a very promising anti-idiotypic monoclonal antibody intended to be used as a therapeutic vaccine against ovarian carcinoma. To MEN2234 the following CA Index Name was assigned: immunoglobulin G1, anti-(mouse OC 125) (mouse monoclonal ACA-125clone 3D5 g-chain), disulfide with mouse monoclonal ACA-125 clone 3D5 k-chain, dimer. The corresponding CAS Registry Number is 792921-10-9. The proposed INN is Abagovomab

The amino acid sequence of MEN 2234 is shown hereinafter.

MEN 2234 is a murine monoclonal antibody generated against the murine monoclonal antibody OC125, that recognises the tumour associated antigen (TAA) CA125. MEN 2234 effectively mimics the tri-dimensional structure of CA125 TAA and so induce in the host the production of anti-anti-idiotypic antibodies (Ab3) targeting the tumour cells expressing the CA125 antigen. MEN 2234 is also able to elicit in the host a cellular immune response specifically directed against CA125 tumour cells.

Approximately 80 % of patients with advanced ovarian cancer have elevated expression of CA125. Although CA125 is over-expressed in ovarian cancer, the human organism itself is not capable to mount an effective immune response against these cancer cells. The treatment with a therapeutic vaccine containing MEN 2234 should instead be able to reawakening the immune system of the host to attack and destroy disseminated ovarian cancer cells.

EP700305 described the monoclonal anti-idiotypic anti-CA125 antibodies, but no specific pharmaceutical formulation suitable for the treatment of pathologies is described.

In Hybridoma, 1995, 14 (2), 164-174, the antibody ACA-125 was characterised, but no formulation was developed.

In Clinical Cancer Research, 2004, 10, 1580-1587 vaccination of patients with ACA125 was performed; no indication on the concentration of aluminium in the formulation was disclosed, but a man skilled in the art, based on prior art, could easily imagine a relatively low concentration of aluminium as adjuvant (lower than 1.25 mg per individual dose).

In Hybridoma, 2005, 133-140, is described a delivery system for continuos endogenous release of ACA125hFc (a chimeric form of ACA 125) to improve its immunogenicity as vaccine against ovarian cancer, but they generated an in vitro depot based on bioencapsulation technology.

Further publication summarises the results of pre-clinical and clinical studies on ovarian cancer vaccines incl. MEN2234 but concrete data on formulation or used concentration of adjuvants are not mentioned (e.g. an aluminium compound is nor cited) : Clinical Cancer Research, 2004, 1580-1587; Clinical Cancer Research, 2003, 3234-3240; Clinical Cancer Research, 2001, 1154-1162; Clinical Cancer Research, 2001, 1112-1115).

### Detailed Description of the Invention

The invention claims a pharmaceutical formulation for parenteral application (preferably subcutaneous or intramuscolar) containing the monoclonal anti-idiotypic antibody MEN2234 adsorbed onto an aluminium compound selected among aluminium hydroxide (alum) or aluminium phosphate, and suspended in an aqueous buffered system.

The use of high concentrations (well above those usually considered and recommended) of an aluminium compound as adjuvant provides a vaccine with unexpected clinical and pharmaceutical advantages such as:
I. guarantee of an extraordinary high specific immunogenicity for both humoral and cellular responses
II. guarantee of a long-lasting induction and maintenance of an immune response
III. guarantee of safety drug administration ;
IV. simplification of manufacturing and release procedure for the final drug product;
V. increased consistency in the manufacturing processes
VI. improvement of drug product stability also at relevant temperature ranges.

Pharmaceutical compositions according to the present invention contain the anti-idiotypic antibody MEN2234 in an amount from 0.1 mg/ml to 4 mg/ml, preferably 0.2mg/ml to 2.5 mg/ml, even more preferably in a concentration approximately of 2 mg/ml (from 1.9 to 2.1 mg/ml). The antibody MEN2234 is adsorbed onto an aluminium compound (preferably at a concentration of approximately 3.5 mg/ml of aluminium) and suspended in a buffered and isotonic saline solution. The present compositions do not need any other ingredient in addition to the antibody adsorbed onto the aluminium compound and the salts of the buffers, resulting in a very simple solution devoid of any additional safety concerns which could derive from the use of other agents such as stabilisers, antioxidants, other adjuvants etc..

Preferred buffers are those obtained with phosphate or citrate salts.

Final composition contains 1 ml of the product to be administered parenterally, preferable via subcutaneous or intramuscular injections.

One of the main characteristic of the present invention is that a relatively high concentration of an aluminium compound, selected among aluminium phosphate and aluminium hydroxide, is required to ensure a nearly complete adsorption of MEN2234 onto the adsorbent; aluminium hydroxide being highly preferred (ref. Table 1).

The preferred concentration of aluminium Al⁺⁺⁺ in the compositions is in the range 2.4-5.2 mg/ml, most preferably approximately 3.5 mg/ml (in the range 3.1-3.8 mg/ml). which corresponds to a content in aluminium hydroxide in the range 0.7 -1.5% W/W, preferably approximately 1% W/W (in the range 0.9-1.1 %)

**Table 1: Dependency of MEN2234 adsorption ( non-adsorbed protein) on aluminium concentration (aluminium hydroxide)**

| **Concentration** | | **Non-adsorbed protein [%]** |
|---|---|---|
| **Al⁺⁺⁺ [mg/ml]** | **Al (OH)₃ [%]** | |
| 0 | 0 | 100 |
| 0.9 | 0.25 | 78 |
| 1.2 | 0.36 | 59 |
| 1.7 | 0.5 | 30 |
| 2.6 | 0.75 | <1.0 |
| 3.5 | 1.0 | <0.1 |
| 6.9 | 2.0 | <0.1 |

The content of aluminium hydroxide 1% corresponds to approx. 3.5 mg aluminium Al⁺⁺⁺ per ml, so well above the content currently used in the registered and marketed vaccines and generally recommended by the authorities.

The present formulations could be prepared according to standard procedures well known in the art. A general procedure that can be used is as follow. The manufacturing of the final product will be performed by mixing the antibody solution and the adjuvant (a gel of aluminium compound) under defined conditions. The drug product has to meet the sterility requirements according to Ph. Eur. Monograph "Parenteral preparation". Consequently, according to the regulatory requirements the manufacturing process will be carried out strictly under aseptic conditions. All used ingredients are sterile grade or are filtered through a 0.22 micrometer filter in form of a solution during manufacturing.

Formulations according to the present application show a consistent group of advantages:

### I. Guarantee of an extraordinary high specific immunogenicity for both humoral and cellular response

We have demonstrated that the monthly treatment (four weekly s.c. administration) of the composition matter of the present invention is able to induce a specific humoral response (specific antibody directed against MEN2234) in an amount dependent on the concentration of the aluminium compound. In fact it has been show that the amount of humoral response elicited by the formulation with 1% aluminium hydroxide (Example1) is significantly superior to that elicited by a suspension containing 0.36 % aluminium hydroxide (corresponding to the limit indicated by Ph.Eur, and FDA guidelines) or by a solution of MEN2234 in buffered saline:

**Table 2: Plasma titers of antibodies against MEN2234 measured in rabbit plasma following 4 weekly s.c. administrations**

| **Formulation** | **Antibody titer (ng/ml)** |
|---|---|
| MEN 2234 solution | 907 ± 259 |
| MEN 2234 suspension in Al(OH)₃ at 0.36 % | 3658 ± 1473 |
| MEN 2234 suspension in Al(OH)₃ at 1.0 % | 15627 ± 5007 * |

| | |
|---|---|
| * P<0.05 vs the other two conditions (Bonferroni Multiple Comparison Test) | |

In addition an histological assessment was carried out at the injection sites to measure the recruitment of inflammatory cells and antigen-presenting cells necessary for the establishment of a specific cellular immune response. Also for this parameter the response to the formulation with 1% aluminium hydroxide (Example 1) was remarkably superior to that achieved with a suspension at lower Al(OH)₃ content (0.36%) or a MEN2234 solution without Al(OH)₃:

**Table 3: Semiquantitative detection of inflammatory cells in the injection sites of rabbit weekly treated with the vaccines**

| **Formulation** | **Density of inflammatory cells** |
|---|---|
| MEN 2234 solution | +/- |
| MEN 2234 suspension in Al(OH)₃ at 0.36 % | ++ |
| M EN 2234 suspension in Al(OH)₃ at 1.0 % | +++ |

### II. Guarantee of a long-lasting induction and maintenance of an immune response

In the rabbit we have demonstrated that following the immunisation (4 weekly injections) with the composition matter in Example 1, the immune response is maintained for a longer time and even after several weeks specific antibodies are measurable in the plasma. On the other hand when the immunisation is done with MEN2234 suspension with a lower amount of Al(OH)3 (0.36 %) or with MEN2234 as a solution without adjuvant, the presence of antibodies is detectable for a shorter period of time:

### III Guarantee of safety drug administration

The MEN2234 suspension matter of this invention has been administered , as s.c. injections at the dose of 2 mg/ml, up to 26 weeks in the rabbit. The scheme of administration was weekly in the first month and then bi-weekly in the remaining period. Local tolerance and systemic safety was assessed. Even after 15 treatments no toxic effects was recorded in terms of: body weight, clinical signs, food consumption, haematology, clinical chemistry, histopathology of all organs. At the site of injections only the inflammatory and immunological response as consequence of the pharmacological action of the drug was observed.

In addition this invention, allowing a reduced amount of free murine antibody, will also decrease its absorption and peak in the systemic circulation thus reducing the risk of anaphylactic reaction(s).

### IV Simplification of manufacturing and release procedure for the final drug product

Approximately % aluminium hydroxide ensures complete MEN2234 binding (<1% non adsorbed antibody). This results in a very robust adsorption and manufacturing process avoiding the need of in-vivo release tests (immunogenic response in experimental animals) as usually required for many aluminium containing vaccines like tetanus, diphtheria. The complete adsorption guarantee an optimal antibody (Ab2) presentation to the host immune system..

Detailed investigations of the MEN2234 - aluminium hydroxide adsorption mechanism revealed that a nearly ideal stoichiometric distribution of both substances is achieved in a phosphate buffer solution. This ensures, as mentioned before, a simple and robust manufacturing process due to the self assembling properties of the invention. Already after 15 sec moderate mixing the antibody is nearly complete adsorbed onto aluminium hydroxide (Table 4).

**Table 4: Dependency of MEN2234 adsorption on mixing time (aluminium hydroxide concentration 1 %)**

| **Mixing time [min:sec]** | **Non-adsorbed protein [%]** |
|---|---|
| 0 | 100 |
| 0:10 | 0.20 |
| 0:30 | 0.20 |
| 1:00 | 0.15 |
| 5:00 | 0.20 |
| 15:00 | 0.03 |
| 240:00 | 0.10 |

As seen in Table1, with concentration of adjuvant minor than 0.75% no complete adsorption could be achieved; concentrations higher than 1% (e.g. 2%) did not show any improvement.

The very quick and complete adsorption of MEN2234 onto aluminium compound at higher concentrations of the adsorbent than recommended allows to remarkably simplify the manufacturing of the composition as a drug. Within seconds the final formulation is obtained simply by mixing the monoclonal antibody with the adjuvant. This mixing step can be performed routinely by using standard equipment for aseptic processing at pharmaceutical facilities. The self-assembling characteristics of the described system leads to completely new perspectives in manufacturing and stabilising of the present formulation as well as for adsorbed monoclonal antibodies or vaccines in general. The complete adsorption of MEN2234 onto the aluminium adjuvant within seconds could also allow the preparation of the final suspension immediately before s.c. or i.m. injection only by mixing the required amounts of drug substance and aluminium adjuvant at the site of final application to patients, e.g. at the hospital.

Therefore a particular aspect of the present invention is represented by a form where the active principle of the vaccine (the antibody MEN2234) and the adjuvant (the aluminium compound, preferably aluminium hydroxide) are kept separated and ready to be mixed just prior of the injection to the patient. The preferred time for this mixing step should be between 10 seconds and 10 minutes before the administration to the patient.

For this particular form, the composition should be provided with the antibody and the adjuvant solution separated each other but filled in pharmaceutical standard container systems like vials, ampoules, pre-filled syringes or appropriate two-chamber systems for mixing and/or reconstitution. The monoclonal antibody can not only be used in form of a eventually buffered solution but also in form of e.g. a powder. The possibility to use the drug substance in solid form allows to apply for it all techniques intended to improve the stability of monoclonal antibodies or vaccines (freeze-drying, spray drying etc.). This represents a significant progress in handling and an improvement in stability for all adsorbed vaccines in general and, in particular, for those containing monoclonal antibodies such as MEN 2234. Main disadvantages of adsorbed vaccines like a permanent cooling chain during shipment and storage, can be avoided and the shelf life of the drug product can be increased.

As mentioned, the in vivo immunogenic activity of monoclonal antibodies containing pharmaceutical compositions depends in a variable manner on the degree of adsorption of the antibody onto the used adsorbents (Capelle et al., Vaccine 2005, 1686 - 1694). Therefore it is state of the art to determine the biological immunogenic activity of (adsorbed) vaccines 'in vivo' in animals prior of the drug product release. The reproducible adsorption of MEN 2234 onto the aluminium adjuvant simplifies the release procedure significantly. Due to the reliability of adsorption, release testing could recourse only on in vitro testing to determine the potency of the composition. The usual procedure to determine also the 'in vivo' biological activity in animals is, for the composition of the present invention, redundant.

### V. Improvement of consistency in drug product manufacturing

The reproducibility in MEN 2234 adsorption using higher concentrations of e.g. aluminium hydroxide than recommended improves significantly the consistency in drug product manufacturing. As demonstrated in Table 5 if the concentration of aluminium hydroxide in the formulations is in the range 0.7 to 2 % w/v the adsorption of MEN 2234 is complete (< 1 % free antibody) for all batches manufactured.

**Table 5: Variability of MEN 2234 adsorption (non-adsorbed antibody) on aluminium hydroxide during manufacturing**

| **Concentration aluminium hydroxide [%]** | **Non-adsorbed antibody [%]** | |
|---|---|---|
| | **Run I** | **Run II** |
| 0 | 100 | 100 |
| 0.25 | 78 | 36 |
| 0.36 | 59 | 11 |
| 0.5 | 30 | 6 |
| 0.75 | < 1.0 | < 1.0 |
| 1.0 | < 0.1 | < 0.1 |
| 2.0 | < 0.1 | < 0.1 |

The complete and reproducible adsorption prevents furthermore feared complications during processing such as the formation of antibody agglomerates. Antibodies in solution are susceptible to form aggregates. Aggregation of antibodies in solution can be induced by excessive shear forces in the liquid phase for example during agitation. These aggregates cannot be re-dissolved and leads to a significant reduction of the biological activity of the vaccine. However, due the complete and rapid adsorption process this phenomenon can be avoided totally for the compositions object of the present invention.

### VI. Improvement of drug product stability also at relevant temperature ranges

Surprisingly, composition of example 1 is by far more stabile (even by 25 °C and 37 °C) in comparison to the buffered MEN2234 solution. This simple but very effective mean (the increase in the aluminium hydroxide concentration up to 2 or 3 fold of the recommended dose) ensures nearly complete binding and accordingly also stability. In Table 6 relevant stability data are presented for MEN2234 in solution and formulated according to example 1.

**Table 6: Stability data for MEN2234 in solution and adsorbed onto aluminium hydroxide**

| **Test product** | **Storage condition [°C]** | **Product [%] after time of** storage **(months)** | | |
|---|---|---|---|---|
| | | **0** | **1** | **2** |
| MEN2234 solution, batch K2CL73-P5 | 25 | 100.0 | 90.2 | 88.6 |
| | 37 | 100.0 | 88.9 | 89.1 |
| MEN2234 adsorbed onto 1 % aluminium hydroxide, batch 287-2p42 | 25 | 100.0 | 102.1 | 98.7 |
| | 37 | 100.0 | 98.7 | 100.4 |

Consequently, the invention ensures the required antibody stability and activity at relevant body temperatures (36 - 37 °C) after administration in humans.

### Examples

Non limiting examples of the invention are the following:

### Example 1: suspension for injection

| Ingredient | mg/ml |
|---|---|
| MEN2234 | 2.00 |
| Al(OH)₃ * | 10.00 |
| KCl | 0.20 |
| KH₂PO₄ | 0.20 |
| NaCl | 8.00 |
| Na₂HPO₄ × 7 H₂O | 2.16 |
| Water for injections | ad 1.00 ml |

| | |
|---|---|
| * used for manufacturing aluminium hydroxide, hydrated, for adsorption The composition was prepared according to standard procedure, as previously described, simply mixing MEN2234 with a suspension of aluminium hydroxide, hydrate, in a solution buffered with the relevant salts. | |

### Example 2: suspension for injection

| Ingredient | mg/ml |
|---|---|
| MEN2234 | 2.00 |
| Al(OH)₃ * | 7.50 |
| KCl | 0.20 |
| KH₂PO₄ | 0.20 |
| NaCl | 8.00 |
| Na₂HPO₄ × 7 H₂O | 2.16 |
| Water for injections | ad 1.00 ml |

| | |
|---|---|
| * used for manufacturing aluminium hydroxide, hydrated, for adsorption Prepared as for example 1 | |

### Example 3: suspension for injection

| Ingredient | mg/ml |
|---|---|
| MEN2234 | 2.00 |
| AlPO₄ | 10.00 |
| KH₂PO₄ | 0.20 |
| NaCl | 9.00 |
| Na₂HPO₄ × 7 H₂O | 1.20 |
| Water for injections | ad 1.00 ml |

### Prepared as for example 1

### Example 4: Amino-acid sequence determination of MEN2234 (abagovomab).

A first part of the amino-acid sequence determination in abagovomab was accomplished using the automatic sequencing techniques according to Edman degradation procedure, in a protein sequencer where abagovomab was covalently linked to the sequencing membrane (first 15 amino acids of the light chain).

As automated Edman sequencing was not able to produce any sequence data of the heavy chain, suggesting a modification of the N-terminal group of the heavy chain to pyroglutamic acid, for a more extended sequencing, the well known LC/MS/MS sequencing techniques were used utilising mass spectrometry ( e. g.. Proc. Natl Acad Sci USA, 1986 Sep Sep, 83(17), 6233-6237).

Briefly, different antibody samples were fragmented with different proteolytic enzymes before LC/MS/MS analysis and yielded several series of overlapping sequence fragments. By finding the overlapping scheme of these fragments, it was possible to determine the total sequence of the light chain of the antibody (complete amino acid sequences of the heavy and light chain are shown in SEQ. ID NO. 1 and SEQ. ID NO. 2).

As a final check for the reliability of the experimental results, the sequences reported were also verified by sequence similarity analysis with the BLAST software package against a non redundant protein database optimized for protein analysis (MSDB, download of March 2007 from the European Bioinformatics Institute ftp site).

Since in mass spectrometry it is impossible to distinguish between isoleucine and leucine, in figure 1 this indecision is represented with "X".

## Claims

1. Pharmaceutical composition for parenteral administration as vaccine comprising a monoclonal antibody and as adjuvant an aluminium derivative in concentration comprised between 2.4 and 5-2 mg/ml aluminium ion wherein:
- said monoclonal antibody is the monoclonal anti idiotypic anti-CA-125 MEN 2234 comprising SEQ. ID NO. 1 and SEQ ID NO. 2 and is present in an amount from 0.1-4 mg/ml and adsorbed onto the aluminium derivative; and
said aluminium compound is chosen among aluminium hydroxide and aluminium phosphate and is suspended in an aqueous buffered system;
**characterised in that** said pharmaceutical composition contains 1 ml of the product to be administered.

2. Pharmaceutical composition according to Claim 1 wherein the aluminium derivative is in concentration comprised between 3.1 - 3.8 mg/ml of aluminium ion.

3. Pharmaceutical composition according to claim 2, wherein the aluminium compound is aluminium hydroxide

4. Pharmaceutical composition according to claims 3 wherein the MEN2234 is present in an amount from 0.2 - 2.5 mg/ml

5. Pharmaceutical composition according to claim 4, wherein MEN2234 is present in an amount from 1.9 to 2.1 mg/ml

6. Pharmaceutical composition according to claims 1-5, wherein MEN2234 is adsorbed onto the aluminium compound and suspended in a buffered and isotonic saline solution.

7. Pharmaceutical composition according to claims 1-6, in the form of a liquid suspension, suitable for parenteral administration.

8. Pharmaceutical composition according to claims 7, suitable for subcutaneous or intramuscolar administration

9. Pharmaceutical composition according to Claims 1 - 8, selected among the following:
a) MEN2234 2.00 mg/ml, Al(OH)₃ 10.00 mg/ml, KCl 0.20 mg/ml, KH₂PO₄ 0.20 mg/ml, NaCl 8.00 mg/ml, Na₂HPO₄ .7 H₂O 2.16 mg/ml, Water for injections up to 1.00 m
b) MEN2234 2.00 mg/ml, Al(OH)₃ 7.50 mg/ml, KCl 0.20 mg/ml, KH₂PO₄ 0.20 mg/ml, NaCl 8.00 mg/ml, Na₂BPO₄.7 H₂O 2.16 mg/ml, Water for injections up to 1.00 m
c) Mean22342.00 mg/ml, AlPO₄ 10.00 mg/ml, KH₂PO₄ 0.20 mg/ml, NaCl 9.00 mg/ml, Na₂HPO₄ x 7 H₂O 1.20 mg/ml, Water for injections ad 1.00 ml

10. Pharmaceutical composition according to claims 1-9 to be used as antitumoral vaccine for the treatment or the prevention of tumours.

11. Pharmaceutical composition according to claim 10 as antitumoral vaccine for the treatment of ovarian tumours.

## Patentansprüche

1. Pharmazeutische Zusammensetzung zur parenteralen Verabreichung als Impfstoff, umfassend einen monoklonalen Antikörper und als Adjuvans ein Aluminiumderivat in Konzentrationen in einem Bereich zwischen 2,4 und 5,2 mg/ml Aluminiumionen, wobei
- genannter monoklonaler Antikörper der monoklonale idiotypische anti-CA-125 MEN 2234 ist, umfassend SEQ. ID NO.1 und SEQ. ID NO.2, und welcher in einer Menge von 0,1 bis 4 mg/ml vorliegt und an dem Aluminiumderivat adsorbiert ist; und
- genannte Aluminiumverbindung ausgewählt ist unter Aluminiumhydroxid und Aluminiumphosphat, welche in einem wässrigen Puffersystem suspendiert ist, und welche **dadurch gekennzeichnet ist, dass** die genannte pharmazeutische Zusammensetzung 1 ml des zu verabreichenden Produkts enthält.

2. Pharmazeutische Zusammensetzung gemäß Anspruch 1, wobei das Aluminiumderivat in Konzentrationen in einem Bereich, umfassend zwischen 3,1 bis 3,8 mg/ml an Aluminiumionen vorliegt.

3. Pharmazeutische Zusammensetzung gemäß Anspruch 2, wobei es sich bei der Aluminiumverbindung um Aluminiumhydroxid handelt.

4. Pharmazeutische Zusammensetzung gemäß Anspruch 3, wobei der MEN2234 in einer Menge von 0,2 bis 2,5 mg/ml vorliegt.

5. Pharmazeutische Zusammensetzung gemäß Anspruch 4, wobei der MEN2234 in einer Menge von 1,9 bis 2,1 mg/ml vorliegt.

6. Pharmazeutische Zusammensetzung gemäß den Ansprüchen 1-5, wobei der MEN2234 an der Aluminiumverbindung adsorbiert ist und in einer gepufferten und isotonischen Salzlösung suspendiert ist.

7. Pharmazeutische Zusammensetzung gemäß den Ansprüchen 1-6 in der Form einer flüssigen Suspension, die für die parenterale Verabreichung geeignet ist.

8. Pharmazeutische Zusammensetzung gemäß Anspruch 7, welche für subkutane und intramuskuläre Verabreichung geeignet ist.

9. Pharmazeutische Zusammensetzung gemäß den Ansprüchen 1-8, welche unter den Folgenden ausgewählt ist:
a.) MEN2234 2,00 mg/ml, Al(OH)₃ 10,00 mg/ml, KCL 0,20 mg/ml, KH₂PO₄ 0,20 mg/mg, NaCl 8,00 mg/ml, Na₂HPO₄ x 7H₂O 2,16 mg/ml, Wasser zur Injektion bis zu 1,00 ml
b.) MEN2234 2,00 mg/ml, Al(OH)₃ 7,50 mg/ml, KCL 0,20 mg/ml, KH₂PO₄ 0,20 mg/mg, NaCl 8,00 mg/ml, Na₂HPO₄ x 7H₂O 2,16 mg/ml, Wasser zur Injektion bis zu 1,00 ml
c.) MEN2234 2,00 mg/ml, A1PO₄ 10,00 mg/ml, KH₂PO₄ 0,20 mg/ml, NaCl 9,00 mg/ml, Na₂HPO₄ x 7H₂O 1,20 mg/ml, Wasser zur Injektion bis zu 1,00 ml

10. Pharmazeutische Zusammensetzung gemäß den Ansprüchen 1-9 zur Verwendung als antitumoraler Impfstoff für die Behandlung oder die Prävention von Tumoren.

11. Pharmazeutische Zusammensetzung gemäß Anspruch 10 als antitumoraler Impfstoff für die Behandlung von Eierstocktumoren.

## Revendications

1. Composition pharmaceutique pour une administration parentérale en tant que vaccin comprenant un anticorps monoclonal et en tant qu'adjuvant un dérivé d'aluminium dans une concentration comprise entre 2,4 et 5,2 mg/ml d'ion aluminium, dans laquelle :
ledit anticorps monoclonal est l'anticorps monoclonal anti-idiotypique anti-CA-125 MEN 2234 comprenant SEQ ID NO : 1 et SEQ ID NO : 2 et est présent dans une quantité de 0,1 à 4 mg/ml et adsorbé sur le dérivé d'aluminium ; et
ledit composé d'aluminium est choisi parmi l'hydroxyde d'aluminium et le phosphate d'aluminium et est mis en suspension dans un système aqueux tamponné ;
**caractérisée en ce que** ladite composition pharmaceutique contient 1 ml du produit à administrer.

2. Composition pharmaceutique selon la revendication 1, dans laquelle le dérivé d'aluminium est dans une concentration comprise entre 3,1 et 3,8 mg/ml d'ion aluminium.

3. Composition pharmaceutique selon la revendication 2, dans laquelle le composé d'aluminium est l'hydroxyde d'aluminium.

4. Composition pharmaceutique selon la revendication 3, dans laquelle le MEN2234 est présent dans une quantité de 0,2 à 2,5 mg/ml.

5. Composition pharmaceutique selon la revendication 4, dans laquelle le MEN2234 est présent dans une quantité de 1,9 à 2,1 mg/ml.

6. Composition pharmaceutique selon les revendications 1 à 5, dans laquelle le MEN2234 est adsorbé sur le composé d'aluminium et mis en suspension dans une solution saline tamponnée et isotonique.

7. Composition pharmaceutique selon les revendications 1 à 6, sous la forme d'une suspension liquide, appropriée pour une administration parentérale.

8. Composition pharmaceutique selon la revendication 7, appropriée pour une administration sous-cutanée ou intramusculaire.

9. Composition pharmaceutique selon les revendications 1 à 8, choisi parmi les suivantes :
a) MEN2234 à 2,00 mg/ml, Al(OH)₃ à 10,00 mg/ml, KCl à 0,20 mg/ml, KH₂PO₄ à 0,20 mg/ml, NaCl à 8,00 mg/ml, Na₂HPO_{4•} 7H₂O à 2,16 mg/ml, eau pour injectable jusqu'à 1,00 ml
b) MEN2234 à 2,00 mg/ml, Al(OH)₃ à 7,50 mg/ml, KCl à 0,20 mg/ml, KH₂PO₄ à 0,20 mg/ml, NaCl à 8,00 mg/ml, Na₂HPO₄• 7H₂O à 2,16 mg/ml, eau pour injectable jusqu'à 1,00 ml
c) MEN2234 à 2,00 mg/ml, AlPO₄ à 10,00 mg/ml, KH₂PO₄ à 0,20 mg/ml, NaCl à 9,00 mg/ml, Na₂HPO_{4•}7H₂O à 1,20 mg/ml, eau pour injectable jusqu'à 1,00 ml.

10. Composition pharmaceutique selon les revendications 1 à 9, à utiliser en tant que vaccin antitumoral pour le traitement ou la prévention de tumeurs.

11. Composition pharmaceutique selon la revendication 10, en tant que vaccin antitumoral pour le traitement de tumeurs ovariennes.
